(19) Europäisches Patentamt
European Patent Office
Office européen des brevets

(11) **EP 4 138 670 B1**

(12) **EUROPEAN PATENT SPECIFICATION**

(45) Date of publication and mention of the grant of the patent:
**05.06.2024 Bulletin 2024/23**

(21) Application number: **21717909.2**

(22) Date of filing: **15.04.2021**

(51) International Patent Classification (IPC):
*A61B 8/08* (2006.01)   *A61B 7/04* (2006.01)

(52) Cooperative Patent Classification (CPC):
**A61B 8/08; A61B 7/001; A61B 7/04; A61B 8/4209; A61B 8/4236;** A61B 8/4245

(86) International application number:
**PCT/EP2021/059748**

(87) International publication number:
**WO 2021/213877 (28.10.2021 Gazette 2021/43)**

(54) **METHODS AND SYSTEMS FOR OBTAINING A MEASUREMENT FROM A SINUS OF A SUBJECT**

VERFAHREN UND SYSTEME ZUR GEWINNUNG EINER MESSUNG AUS EINEM SINUS EINES SUBJEKTS

PROCÉDÉS ET SYSTÈMES PERMETTANT D'OBTENIR UNE MESURE D'UN SINUS D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2020 EP 20170441**

(43) Date of publication of application:
**01.03.2023 Bulletin 2023/09**

(73) Proprietor: **Koninklijke Philips N.V.**
**5656 AG Eindhoven (NL)**

(72) Inventors:
• **HIWALE, Sujitkumar**
**5656 AE Eindhoven (NL)**
• **CHAKRABARTI, Biswaroop**
**5656 AE Eindhoven (NL)**
• **BERA, Deep**
**5656 AE Eindhoven (NL)**
• **JOHNSON, Mark, Thomas**
**5656 AE Eindhoven (NL)**

(74) Representative: **Philips Intellectual Property & Standards**
**High Tech Campus 52**
**5656 AG Eindhoven (NL)**

(56) References cited:
EP-A1- 1 391 178   WO-A1-2018/096335
SU-A1- 1 648 379   US-A1- 2004 210 135
US-A1- 2005 154 301

**Description**

FIELD OF THE INVENTION

The invention relates to the field of sinus investigation, and more specifically to the field of acoustic sinus investigation.

BACKGROUND OF THE INVENTION

Infection of paranasal sinuses is known as sinusitis, which can vary in severity from mild to distressing and debilitating. Sinusitis is usually self-limiting, however in some cases it can become chronic, negatively impacting the quality of life. It has been estimated that sinusitis contributes to more than 3% of all patient visits to primary care and emergency medical departments, with chronic sinusitis substantially more common than acute sinusitis as discussed in J. Novis et al, "A Diagnostic Dilemma: Chronic Sinusitis Diagnosed by Non-Otolaryngologists," Int Forum Allergy Rhinol, vol. 6, no. 5, pp. 486-490, May 2016.

Sinusitis along with other acute upper respiratory tract infections (URTIs) are a major cause of lost days of work and schooling. The economic impact of the common cold alone on workplace absenteeism is estimated to be billions of dollars as found in J. Bramley et al, "Productivity losses related to the common cold," J. Occup. Environ. Med., vol. 44, no. 9, pp. 822-829, Sep. 2002.

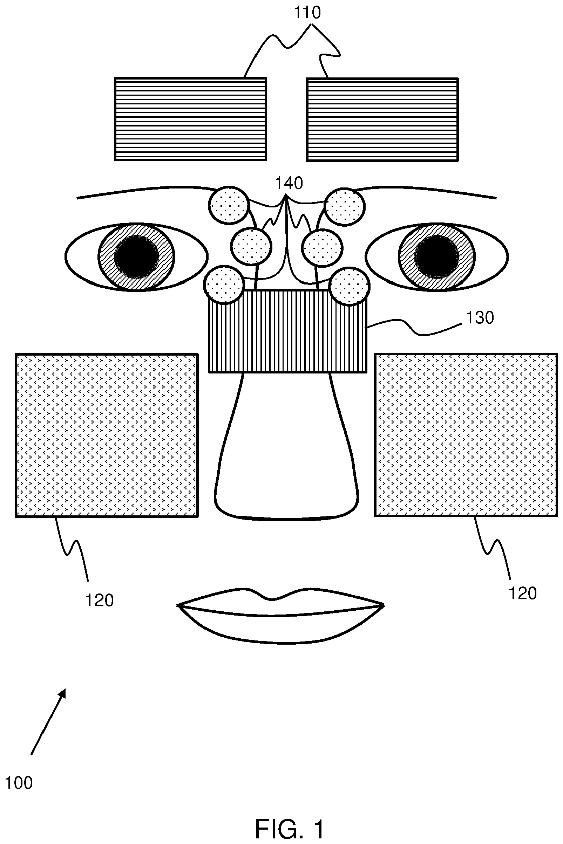

The paranasal sinuses are air-filled spaces around the nasal cavity present within the skull bones. The paranasal sinuses include four different groups: the frontal sinuses; the maxillary sinuses; the sphenoidal sinus; and the ethmoidal sinuses. Figure 1 shows a schematic representation 100 of the paranasal sinuses present in the human head. The frontal sinuses 110 are typically located on the brow above the eyes and are roughly symmetrical along a line defined by the center of the face. The maxillary sinuses 120 are typically located in the cheekbones below the eyes and are roughly symmetrical along a line defined by the center of the face. The sphenoidal sinus 130 is typically located at the rear of the nasal cavity and is roughly in line with the ears. The ethmoidal sinuses 140 are typically at the top of the nasal cavity and are located roughly in line with the eyes.

These sinuses open through small openings, referred to as ostia, on the lateral wall of the nasal cavity. The primary function of sinuses is to make the skull lighter and to add resonance to the voice. Sinuses are lined by a mucous membrane, the mucus being swept from the sinuses to the nasal cavity through the ostia by cilia. Blockage of ostia due to inflammation in the nose causing retention of mucous is the presumed mechanism for sinus infections, i.e. sinusitis.

Sinusitis is usually diagnosed based on clinical history and physical examination. Common symptoms include thick nasal discharge, a blocked nose, headaches, decreased sense of smell and taste and facial pain. Physical examination for sinusitis involves palpation of bony surface over sinus, checking for numbness, pain, swelling, and/or firmness on face and the lymph nodes in the neck area. However, the symptoms of sinusitis overlap with other common conditions, including allergic rhinitis, viral upper respiratory tract infection, deviated nasal septum, and migraine headaches. For this reason, a clinical diagnosis of sinusitis is often inaccurate, especially in cases of chronic sinusitis as discussed in N. Bhattacharyya, "Clinical and symptom criteria for the accurate diagnosis of chronic rhinosinusitis," Laryngoscope, vol. 116, no. 7 Pt 2 Suppl 110, pp. 1-22, Jul. 2006. Inaccurate diagnosis of sinusitis can lead to unnecessary antibiotic treatment in primary care, which is undesirable.

Physical examination of sinusitis is typically followed by radiograph, CT or magnetic resonance imaging (MRI) investigation, if required. If clinician suspects a sinus tumor then CT is usually performed to visualize a subject's paranasal sinus cavities. Although CT scans are very helpful in sinus diagnosis, it is not recommended in OPD setup to avoid unnecessary ionizing radiation to subjects, and in particular pregnant women. Researchers have also investigated the validity of ultrasonography compared with radiography and MRI in detection of maxillary sinusitis. It has been observed that ultrasound technique has high specificity compared to MRI; therefore, a positive ultrasound finding can be regarded as evidence of maxillary sinusitis as shown in T. Puhakka et al., "Validity of Ultrasonography in Diagnosis of Acute Maxillary Sinusitis," Arch Otolaryngol Head Neck Surg, vol. 126, no. 12, pp. 1482-1486, Dec. 2000. Although, ultrasound-based devices can detect infections in some sinuses, they are typically limited in detecting infections deep within a sinus.

There is therefore a need for a means of accurately investigating any sinus of a subject without significant hardware requirements or causing risk or discomfort to the subject.

Document US 2005/154301 discloses a system for diagnosing sinusitis in a human subject including: a miniature transducer for emitting a sound signal; a miniature microphone for detecting sound signals, each contained in a holder adapted to be inserted into a nostril of the subject.

The system disclosed in US 2005/154301 requires the insertion of both a transducer and microphone into the nostrils of a subject in order to obtain acoustic signals from the sinuses of the subject, which may cause discomfort to the subject, particularly in pediatric applications.

Document RU 169125 discloses a system using external tone sources for investigating a frontal sinus of subject.

The system described in RU 169125 is only suitable for investigating a frontal sinus and requires the use of external vibration sources.

Document SU 1648379 discloses a method for investigating frontal sinuses only and requires the insertion of multiple optical fibers into the frontal sinuses of the subject, which are then investigated by way of an external vibration source, such as a transducer, applied to the soft tissues of the head.

The methods described in SU 1648379 are only suitable for investigating a frontal sinus and require the invasive insertion of optical fibers into the frontal sinuses in order to position the external vibration source.

US 2004/0210135 discloses a diagnostic ultrasound system using shear waves. One use case described is to detect fluid in a patient's sinus cavity.

SUMMARY OF THE INVENTION

The invention is defined by the claims.

According to examples in accordance with an aspect of the invention, there is provided a device for detecting potential sinus pathologies, comprising:

a patch, comprising one or more vibration sensing devices, configured to be placed on facial bones overlying a sinus of a subject, wherein the patch is configured to obtain facial bone vibration signals generated due to vibrations in facial bones surrounding the sinus, wherein characteristics of the facial bone vibration signals are representative of condition of the sinus; and

a processing subsystem operationally coupled to the patch and configured to:

receive the facial bone vibration signals from the patch; and

determine a physiological measure of the sinus of the subject based on characteristics of the facial bone vibration signals.

The vibrations in the facial bones forming the sinus are generated due to vocalization of sounds by the subject resulting in resonant frequency vibrations of the facial bones, wherein the facial bone vibration signals comprise the resonant frequency vibrations.

By utilizing facial bone vibration signals generated by way of the subject making sounds, it is ensured that any sinus undergoing investigation will be fully insonified, thereby increasing the accuracy of the subject sinus vibration signal to be used in the comparison.

By providing a sensor within a patch to be applied to the face of the subject, the plurality of facial bone vibration signals may be acquired without requiring the sensor to be inserted into the subject, such as within a nostril. In this way, the sensor may be positioned at any desired point on the face of the subject in order to investigate the sinus of interest.

The device provides a means of obtaining a physiological measure of a sinus of a subject in an accurate manner that is comfortable to the subject.

The patch may be applied with an adhesive or it may be fixed in by surface tension forces. The patch for example comprises a closed shape on which the vibration sensing devices are mounted. As a minimum, there is one vibration sensing device (which may be sequentially applied to different positions), but preferably the patch holds a set of vibration sensing devices such as between 3 and 20 such sensors. Thus, the sensing can be performed in parallel.

The patch for example comprises a forehead portion, a cheek portion and a narrower nose bridge portion for extending along the nose bridge between the eyes, and joining the forehead portion and the cheek portion.

In an embodiment, the patch additionally comprises a vibrator configured to vibrate the facial bones at a frequency resulting in resonant frequency vibrations of the facial bones, wherein the facial bone vibration signals comprise the resonant frequency vibrations.

When provided to a sinus of the subject, the resonant frequency of the sinus in question will result in the largest intensity and duration of bone vibration, thereby resulting in in facial bone vibration signals of greater intensity. In this way, it is ensured that the facial bone vibration signals will be well defined, thereby increasing the accuracy of the derived physiological measure of the sinus.

In an embodiment, a size and a shape of the patch is dependent on an age, geographical region, target category of sinus, or combinations thereof.

In this way, the patch may be adapted to fit any individual subject.

In an embodiment, the processing subsystem is configured to determine the physiological measure of the sinus by:

obtaining a plurality of reference sinus vibration signals corresponding to a plurality of categories of sinuses, wherein a reference sinus vibration signal corresponding to a category of sinus is different from another reference sinus vibration signal corresponding to another category of sinus;
comparing the facial bone vibration signals to the reference sinus vibration signals; and
deriving a physiological measure of the sinus of the subject based on the comparison.

Sinuses possess characteristic frequency responses that change based on the shape and physiological status of the sinus, for example inflammation of the sinus lining. By comparing the facial bone vibration signals to the reference sinus vibration signals, a physiological measure of the sinus may be derived based on a disparity between the signals.
In an embodiment, the patch is configured to obtain the facial bone vibration signals comprising a first plurality of facial bone vibration signals from a left side of the face of the subject and a second plurality of facial bone vibration signals from a right side of the face of the subject, and wherein the processing subsystem is further configured to:

compare the first plurality of facial bone vibration signals to the second plurality of facial bone vibration signals;
determine a change in sinus resonance between the left side of the face and the right side of the face based on the comparison; and
if the change in sinus resonance exceeds a predetermined threshold, indicating the presence of an asymmetrical physiological sinus status.

In this way, a sinus requiring additional investigation may be readily identified based on the presence of an asymmetrical signal response between the left and right sides of the face of the subject.
In an embodiment, the one or more vibration sensing devices comprises an inertia measurement unit (IMU) sensor, a microphone, an optical based sensor, or a combination thereof.
In an embodiment, the processing subsystem is further configured to determine a category of sinus based on the resonant frequency of the facial bone vibration signals.
In this way, the type of sinus undergoing investigation may be identified.
In an embodiment, the patch is configured to obtain the facial bone vibration signals comprising a third plurality of facial bone vibration signals acquired when the head of the subject is maintained in an upright position and a fourth plurality of facial bone vibration signals acquired when the head of the subject is maintained at an angle to the upright position, and wherein the processing subsystem is further configured to:

compare the third plurality of facial bone vibration signals to the fourth plurality of facial bone vibration signals; and
derive a further physiological measure of the sinus of the subject based on the comparison.

In this way, additional measures relating to the physiological status of the sinus may be derived, such as the behavior of a fluid within a sinus.
In an embodiment, the patch further comprises a positioning sensor adapted to determine a position of the patch on the face of the subject, and wherein the system further comprises a user interface, wherein the processor is adapted to generate a user guidance signal for adjusting a position of the patch based on the position determined by the positioning sensor.
In this way, the positioning of the patch may be guided in order to position the patch at an optimal location.
According to examples in accordance with another aspect of the invention, there is provided a computer-implemented method for determining a physiological measure of a sinus of a subject, wherein said physiological measure may be used for detecting potential sinus pathologies, the method comprising

obtaining facial bone vibration signals from a patch, comprising one or more vibration sensing devices placed on facial bones overlying the sinus of the subject, the facial bone vibration signals being generated due to vibrations in facial bones surrounding the sinus and generated due to vocalization of sounds by the subject resulting in resonant frequency vibrations of the facial bones, wherein characteristics of the facial bone vibration signals are representative of condition of the sinus and the facial bone vibration signals comprise the resonant frequency vibrations;
and determining, using a processing subsystem operationally coupled to the patch, a physiological measure of the sinus of the subject based on characteristics of the facial bone vibration signals.

In an embodiment, the method further comprises:

obtaining the facial bone vibration signals comprising a plurality of a first plurality of facial bone vibration signals from a left side of the face of the subject and a second plurality of facial bone vibration signals from a right side of the face of the subject;
comparing the first plurality of facial bone vibration signals to the second plurality of facial bone vibration signals;
determining a change in sinus resonance between the left side of the face and the right side of the face based on the comparison; and
if the change in sinus resonance exceeds a predetermined threshold, indicating the presence of an asymmetrical physiological sinus status.

In an embodiment, the method further comprises:

obtaining the facial bone vibration signals comprising a plurality of a third plurality of facial bone vibration signals acquired when the head of the subject is maintained in an upright position and a fourth plurality of facial bone vibration signals acquired when the head of the subject is maintained at an angle to the upright position;
comparing the third plurality of facial bone vibration signals to the fourth plurality of facial bone vibration signals; and
deriving a further physiological measure of the sinus of the subject based on the comparison.

According to examples in accordance with another aspect of the invention, there is provided a computer program comprising computer program code means which is adapted, when said computer program is run on a computer, to implement the methods described above.
These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a schematic representation of the head of a subject;
Figure 2 shows method of the invention;
Figure 3 shows a schematic representation of a patch according to an aspect of the invention attached to the face of a subject
Figure 4 shows a method for determining whether a sinus ostium is open or blocked; and
Figure 5 shows a method for determining a physiological measure of a sinus.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention as defined by the appended claims. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a device for detecting potential sinus pathologies, comprising a patch, comprising one or more vibration sensing devices, configured to be placed on facial bones overlying a sinus of a subject, wherein the patch is configured to obtain facial bone vibration signals generated due to vibrations in facial bones surrounding the sinus. The characteristics of the facial bone vibration signals are representative of condition of the sinus. The device further comprises a processing subsystem operationally coupled to the patch and configured to receive the facial bone vibration signals from the patch and determine a physiological measure of the sinus of the subject based on characteristics of the facial bone vibration signals.
Paranasal sinuses, and their acoustic properties, play a significant role in forming natural nasal resonance of the voice. A sinus maybe modelled as a Helmholtz resonator to approximate the sinus' contribution in speech. A sinus being a closed cavity of known dimension with a narrow neck and small opening corresponds very closely to an ideal Helmholtz resonator. Helmholtz resonance is the phenomenon of air resonance in a cavity, and by modelling a Helmholtz resonator it is possible to study the contribution of a sinus in nasal resonance.
The resonant frequency of a Helmholtz resonator is determined by the volume, $V$, of the cavity and the dimensions of the neck, which are determined by the cross-sectional area, $A$, and the effective length, $l_e$, of the neck. The Helmholtz resonator stipulates that both the diameter '$A$' and length '$l_e$' of the resonator's neck must be small compared to the wavelength of the sound. In general, the paranasal sinus ostia meet this condition. With a series loss, $R$, and a shunt loss, $G$, the fundamental resonant frequency ($f$) of a Helmholtz resonator is given by the following formula:

$$f = \frac{c}{2\pi} \sqrt{\frac{1+RG}{LC} - \frac{1}{4}\left(\frac{R}{L} + \frac{G}{C}\right)^2}, \quad (1)$$

where: $L = l_e/A$; $C = V/\rho c^2$; $\rho$ is the density of air; and c is the velocity of sound in air.

For a particular patient the actual dimensions of the paranasal sinuses may not be available; however, usable approximations may be calculated from simple biometric, such as: head circumference; or demographic information such as race, gender, age, height and the like. Typical antiresonance frequency ranges of different sinuses have been observed using morphologic data and experimental studies, involving analysis of speech by microphones placed in the nasal cavity or in front of nose or mouth. The recorded signal is then used to study antiresonance frequency ranges of different sinuses. The antiresonance frequency of different sinuses have been found to be in the range of 200 Hz to 4 kHz, as discussed in M. Havel et al, "Eliminating paranasal sinus resonance and its effects on acoustic properties of the nasal tract," Logoped Phoniatr Vocol, vol. 41, no. 1, pp. 33-40, 2016.

From the Equation (1), it is clear that in addition to the volume of paranasal sinuses, the dimensions of an ostia ($l_e$ and $A$) are also critical factors in the determination of the resonant frequency of a sinus. Therefore, blockage of a sinus ostia (as is often the case in sinusitis) is likely to change the resonant frequency of a sinus. characteristic dips in nasal resonance for the different sinuses have been observed in studies of nasal sounds under conditions of open and blocked sinus ostia. For example, for the maxillary sinuses the characteristic dip has been observed at 400 - 1000 Hz, as discussed in T. KOYAMA, "Experimental study on the resonance of paranasal sinus," Nippon Jibiinkoka Gakkai Kaiho, vol. 69, no. 6, pp. 1177-1191, 1966.

The term insonify used herein refers to the flooding of an area or an object with controlled sound waves. Put another way, the term insonify refers to the provision of sound waves to an area of interest, such as a sinus.

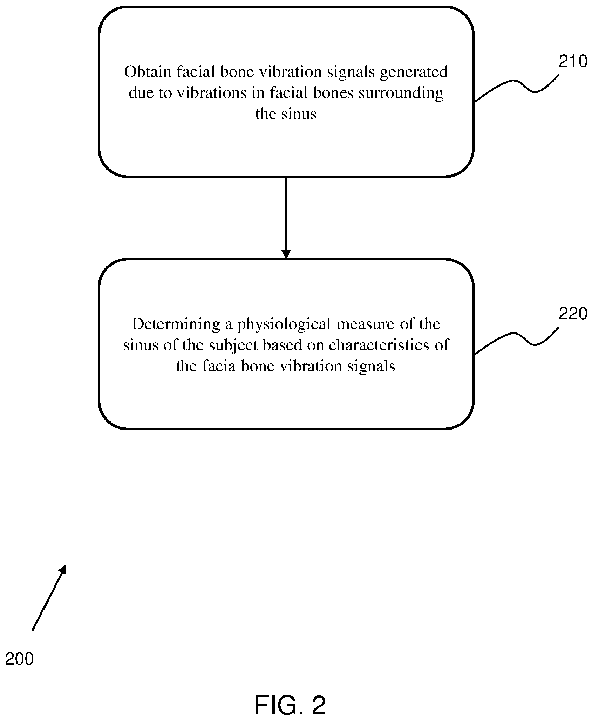

Figure 2 shows method 200 for deriving a physiological measure of a sinus of a subj ect.

The method begins in step 210 by obtaining facial bone vibration signals generated due to vibrations in facial bones surrounding the sinus, wherein characteristics of the facial bone vibration signals are representative of condition of the sinus.

In step 220, a physiological measure of the sinus of the subject is determined based on characteristics of the facial bone vibration signals.

The physiological measure of the sinus may be determined by comparing the facial bone vibration signals to reference sinus vibration signals. The plurality of reference sinus vibration signals may be obtained from any suitable source. For example, the plurality of reference sinus vibration signals may be obtained from a sinus model, such as a sinus modelled as a Helmholtz resonator using Equation (1) as discussed above. Alternatively, or in addition, a database of subject sinus vibration data, collected from a plurality of healthy subjects, may be used to provide average values to be used as reference sinus vibration signals.

The reference sinus vibration signals may include a reference resonant frequency of a reference sinus. For example, the reference sinus vibration signals for a frontal sinus may include the resonant frequency, or the typical range of resonant frequencies, for a healthy frontal sinus.

Variations of Equation (1) have been used in the literature to study resonance properties of a sinus under normal conditions. However, the inventors have recognized that Equation (1) may be used to estimate acoustic properties of a sinus during sinusitis as well. During sinusitis, a sinus becomes filled with mucous. With mucous collection, the volume ($V$) and properties of the air ($\rho$) inside the cavity of sinus changes, resulting in change in resonance frequencies. For different levels and different types of fluid (serous (watery) discharge or thick discharge) the changes in resonant frequencies may be calculated.

Further, Nitric Oxide metabolites are significantly higher in sinuses of patients with chronic sinusitis as discussed in M. Naraghi et al, "Nitric oxide: a new concept in chronic sinusitis pathogenesis," Am J Otolaryngol, vol. 28, no. 5, pp. 334-337, Oct. 2007. As Nitric Oxide is denser than atmospheric air, it is likely that the overall air density ($\rho$) inside the nasal cavity is be altered. Therefore, the changes in resonant frequency of a sinus for different concentrations of Nitric Oxide may be similarly calculated.

Two additional factors that have bearing on the resonance frequencies are series loss, $R$, and shunt loss, $G$. $R$ is approximated by the viscous resistance of the neck and $G$ consists of sinus wall vibration conduction and heat conduction. The sinus wall consists of a bony wall with a thin layer of mucous membrane of 0.02 to 0.08 cm in thickness. As the bony portion of a sinus wall does not change much in adult life, the properties of the mucous membrane, and in particular the thickness and composition of the mucus membrane are important factors, which dictate shunt loss, $G$. As $G$ is directly proportional to resonant frequency, any change in $G$ will lead to a proportionate change in resonant frequency. During chronic sinusitis, the mucous membrane is inflamed and is thicker than normal sinus mucosa leading to a change in $G$ and the resonant frequency of a chronically inflamed sinus.

Based on Equation (1), reference charts of resonant frequencies may be created for each of the sinuses for normal and pathological conditions. For example, it can be seen from the Equation (1), that the volume ($V$) of the sinus in inversely proportional to the fundamental frequency, meaning any decrease in volume (as in case of sinusitis with mucus accumulation in the sinus cavity) will lead to a proportional increase in the resonant frequency compared to that of a sinus without any mucous accumulation. Put another way, for different volumes of fluid, the resonant frequency of a sinus will change in a predictable manner. Based on this information, experimental data may be generated to link fluid volume in a sinus to a corresponding resonant frequency (or change in the resonant frequency) of the sinus.

In addition to the accumulation of mucous (and associated decrease in volume of the sinus), the cases of chronic sinusitis are also likely to have high Nitric Oxide inside the sinus, leading to an increase in $\rho$, which is likely to further increase the resonant frequency of the sinus. Similar, reference charts may be generated for various levels of Nitric Oxide, shunt loss, G, and the resonant frequency of a sinus.

Similarly, a change in resonant frequency of a sinus as a result of any other pathological condition, such as: sinus polyps; a sinus tumor; and the like, may be also incorporated into a reference database for associated changes in resonant frequency.

The plurality of facial bone vibrations may be acquired by any suitable means. The subject signals may be acquired directly from the subject at the same time as the current method is being performed. Alternatively, the subject signal may be obtained from a database having been acquired prior to the current method.

As discussed above, the paranasal sinuses are air-filled spaces within the skull bones. Therefore, any vibrations within a sinus also get transmitted to the bones forming walls of the sinus. It has been observed that different sounds have different impact on magnitude of facial bone vibration and it is possible to reliably measure the extent of bone vibration in relation to resonant voice. As it is not possible to determine resonance frequency of a sinus directly, the facial bone vibration information can be used to determine a resonance frequency of a sinus underlying the facial bones.

The facial bone vibration signals may be acquired from a subject generating a reference sound. In other words, the subject produces a reference sound, or vocalization, in order to cause the bones of the skull, and so the sinuses, to vibrate. Put another way, the subject vocalizes a sound at a reference frequency, or across a range of reference frequencies, thereby insonifying all of the sinuses simultaneously without the need for extensive external vibration sources that would otherwise be required. The facial bone vibration signals may comprise a resonant frequency of the sinus of the subject, which may then be compared to the reference resonant frequency as described above.

As the subject is generating the reference sound and so insonifying all of the sinuses within the skull, it is possible to investigate any sinus in order to derive a physiological parameter of interest. Thus, the facial bone vibration signals are received from the patch during vocalization of sounds by the subject, resulting in the resonant frequency vibrations of the facial bones. For example, the sinus may be: a frontal sinus, wherein the plurality of reference sinus vibration signals comprise a frontal sinus reference signal; a maxillary sinus, wherein the plurality of reference sinus vibration signals comprise a maxillary sinus reference signal; a sphenoidal sinus, wherein the plurality of reference sinus vibration signals comprise a sphenoidal sinus reference signal; and an ethmoidal sinus, wherein the plurality of reference sinus vibration signals comprise an ethmoidal sinus reference signal. These are different categories of sinus.

By comparing the facial bone vibration signals obtained from the subject to the reference sinus vibration signals described above, the condition of the sinus of the subject may be derived. The reference signals may be used to derive thresholds or train machine learning models to detect various physiological measures of the sinuses based on the comparison result.

The physiological measure may be any measure derivable from the comparison between the subject signals and the reference signals, such as mucus volume within a sinus or sinus ostium blockage.

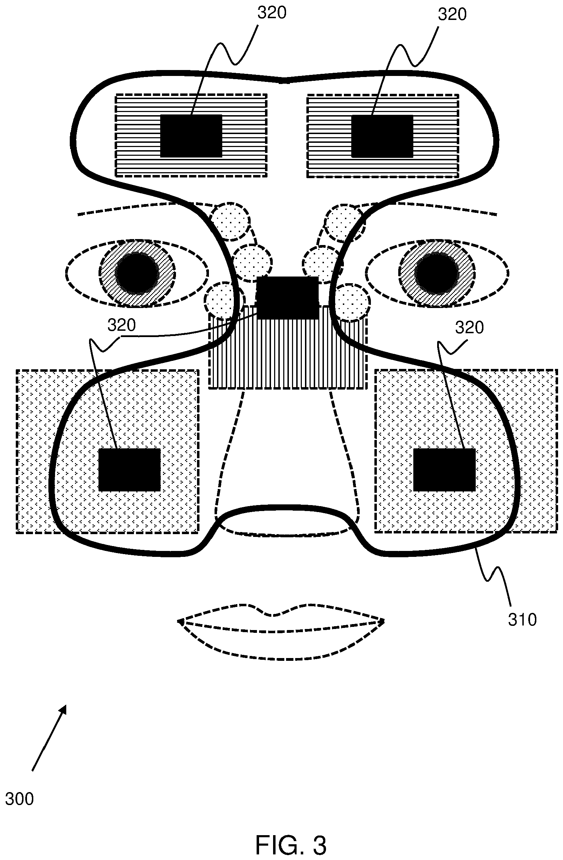

The facial bone vibration signals are acquired by way of a patch positioned on the face of a subject. Figure 3 shows a device 300 according to an aspect of the invention, comprising a patch 310 attached to the face of a subject. The patch 310 comprises a plurality of sensors 320 adapted to detect vibrations from a sinus region of the subject. The number of sensors may be adapted according to the implementation of the patch. The sensor may be any sensor capable of detecting a vibration, such as: a microphone or an accelerometer. The sensors may be mirrored for the left hand side of the face and the right hand side of the face.

In this example, the patch comprises a closed shape on which the vibration sensing devices are mounted. The patch comprises a forehead portion extending across both sides of the forehead above the eyes, a cheek portion extending over the nose and to both sides of the nose, and a narrower nose bridge portion extending along the upper nose bridge between the eyes. In this way, the patch covers the facial bones of interest.

The patch may further comprise a processor adapted to carry out the methods described above and below, such as a processing unit, a microcontroller and the like. The patch may further comprise a means of communicating with an external processor adapted to carry out the methods described above and below, such as a wireless or wired communication means. The processor may be any processing system suitable for carrying out the methods described above or below. The external processor may comprise a computer, a laptop, a smartphone, a smart device, a distributed processing system, cloud-based processing system and the like.

During use, the patch 310 may be placed over the face of the subject, such that it covers the nasal bones and underlying sinuses as shown in Figure 3. The patch may then be used to record acoustic properties of a sinus by recording vibrations of bones overlying the sinus as the subject generates a reference sound. The received vibration signals are then analyzed as described above and compared with reference signals in order to derive a physiological measure of the sinus of interest. The position of the sensors may be adjustable for different age groups to account the variation in the sinus anatomy area in frontal, ethmoidal, and maxillary locations.

Alternatively, a patch comprising a single sensor may be used sequentially over different facial bones and the received vibration signals processed to generate a vibration map of entire face.

The patch may further comprise an actuator adapted to insonify a sinus region of the subject. In such an example, the method described above with respect to Figure 2 may further comprise the step of obtaining a plurality of induced sinus vibration signals, wherein the induced sinus vibration signals have been acquired from a sinus of a subject undergoing insonification from a source external to the subject, the external source being the actuator in the patch.

The actuator may be adapted to generate vibrations of a specific desired frequency, such as a reference resonance frequency of the sinus for the purposes of supplementing the sound generated by the subject.

Furthermore, not all sinus positions require their own actuator. For example, a centrally positioned actuator may be used for assessing the ethmoidal sinuses on both the left side and the right side of the nasal cavity. Furthermore, the sensors and actuators of the patch may be used to assist patch positioning. Alternatively, further dedicated patch positioning sensors, for example using body channel positioning principles, may also be incorporated into the patch.

In other words, the patch may further comprise a positioning sensor adapted to determine a position of the patch on the face of the subject, and wherein the system further comprises a user interface, wherein the processor is adapted to generate a user guidance signal for adjusting a position of the patch based on the position determined by the positioning sensor.

In addition to the sensors, and in some cases actuators, the system may include one or more separate vibration sensors and actuators that are not integrated on a patch. The separate actuators may be positioned on predefined points corresponding to the sinus anatomy and positioning of the separate sensors varied to obtain vibration measurements from multiple points. These signals may then be used to supplement the facial bone vibration signals.

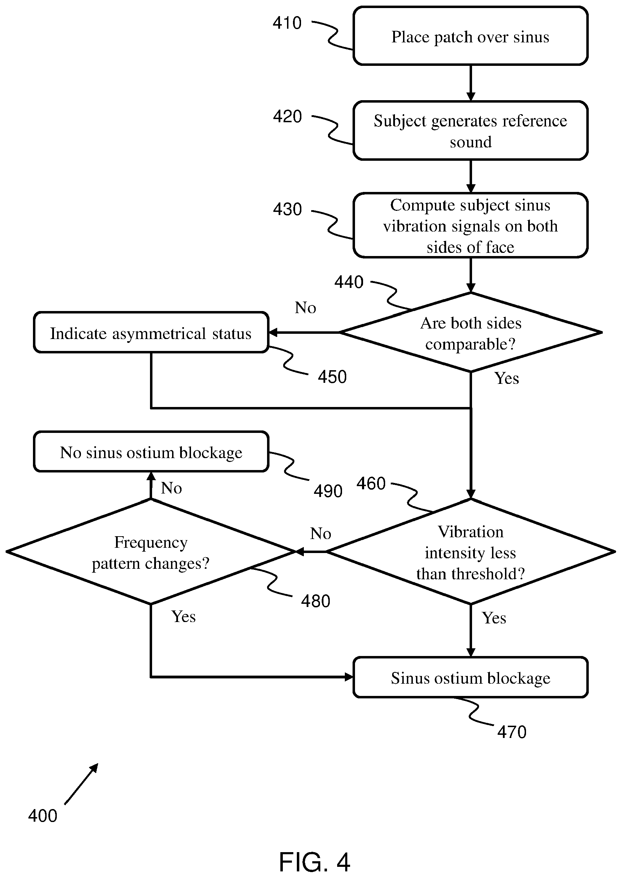

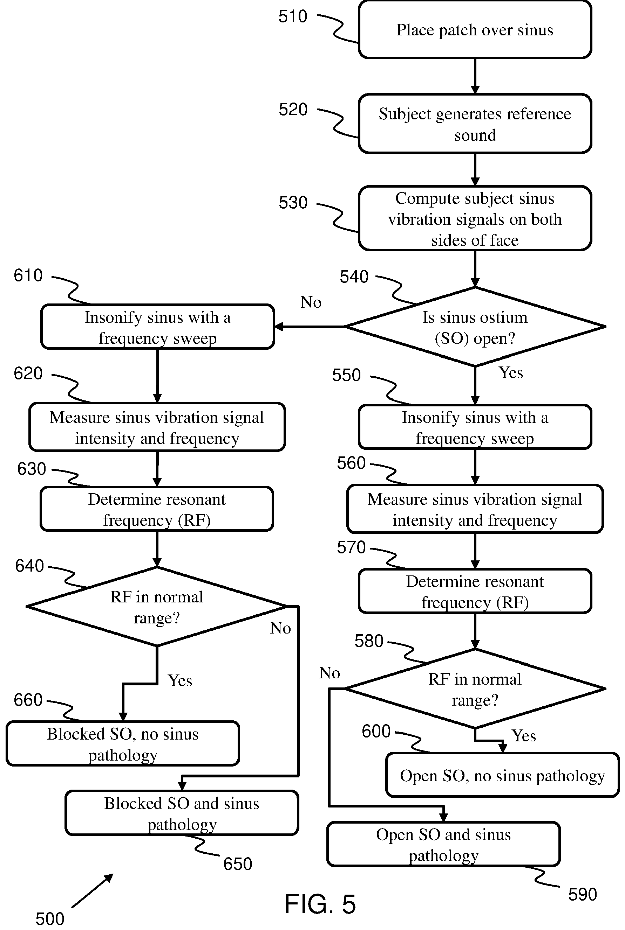

Several use cases of the method described with reference to Figure 2 and the patch shown in Figure 3 are described below with reference to Figures 4 and 5.

Figure 4 shows a method 400 for determining whether a sinus ostium is open or blocked. The presence of a sinus ostium blockage may form part of the physiological measure derived in the method described above with reference to Figure 2.

The method begins in step 410 by positing a patch, such as the patch described above with reference to Figure 3, over the face of the subject covering the bones that form the walls of the sinuses.

To place the patch, anatomical knowledge related to the layout of the sinuses may be used. For example, for maxillary sinuses, bone vibration during resonant voice production is generally considered to be prominent at the maxillary bones as discussed in F. C. Chen et al, "Facial bone vibration in resonant voice production," J Voice, vol. 28, no. 5, pp. 596-602, Sep. 2014. Further, bone vibration due to resonant voice production may be at a maximum at the forehead (for frontal sinuses) at approximately 1 cm above the midline of the eyebrows (glabella) and the nasal bridge bone (medial or inferomedial to the zygomatic arch) just above the septal cartilage (for ethmoidal sinuses) as discussed in E. M.-L. Yiu et al, "Vibratory and perceptual measurement of resonant voice," J Voice, vol. 26, no. 5, pp. 675.e13-19, Sep. 2012.

Alternatively, as each sinus possess a characteristic vibration intensity and frequency pattern during nasal resonance, this information may be used to detect a sinus underlying the patch, which may then be used to adjust the position of the patch.

In step 420, the subject is asked to speak words with specific nasal consonants, such as M or N, or to hum in order to generate the reference sound. The patch in used to record facial bone/skin vibration during phonation by the subject. Different sounds have different impacts on the magnitude of facial bone vibrations, meaning a map can be produced for different nasal consonants and their respective facial bone/skin vibration patterns.

In step 430, the facial bone vibration signals are calculated for both sides of the subject's face based on the vibration signals detected by the patch.

In step 440, the vibration signals from the two sides of the face are compared to determine the presence of any characteristic changes in the nasal resonance pattern from either side of the face. If a clear change is detected, such as a strong asymmetry in the resonance dip from 400-1000Hz associated with the maxillary sinus, is observed the method proceeds to step 450 where the asymmetrical status of the subject's sinuses are noted. Put another way, in step 450, a potential pathology in the sinuses is indicated as requiring further investigation. Further, the side of the face requiring further investigation may also be indicated.

The subject sinus vibrations signals are then compared to threshold values in step 460. For example, the facial bone vibration signals may be compared to the map of nasal consonants and their respective facial bone/skin vibration pattern under normal and pathological conditions. When a particular sinus ostium is blocked, there will be no transmission of voice into the given sinus, meaning the sinus will not resonate during voice production. This will result in reduced vibration of bones/skin overlying this sinus. Therefore, for each sinus a threshold can be also computed for vibration intensity; a value above this threshold would indicate open sinus, whereas the value below the threshold would indicate ostium blockage.

If the vibration intensity the facial bone vibration signals is below a predefined threshold for a particular sinus, the method progresses to step 470, in which it is determined that the sinus ostium is blocked.

If the vibration intensity the facial bone vibration signals is above the predetermined threshold, the method progresses to step 480, wherein the frequency patterns of the facial bone vibration signals may be compared to reference sinus vibration signals. The presence of characteristic dips or patterns within a particular frequency range may indicate the blockage of a particular nasal sinus ostium, in which case the method progresses to step 470, in which it is determined that the sinus ostium is blocked. Otherwise. the method progresses to step 490, in which it is determined that the sinus ostium is not blocked.

Alternatively, the vibration characteristics in the nasal bones (rather than the bones over a particular sinus) may be used for detecting sinus ostium blockage. The nasal bones, and in particular the lateral bones/cartilage on either side of the nasal bridge, serve as a sensitive area for the detection of vibrations during nasal resonance.

Accordingly, the patch described above with respect to Figure 3, or a portion of said patch, may be placed over the face of the subject such that it covers the nasal bones, and in particular the lateral bones/cartilage on either side of the nasal bridge.

The subject is asked to speak words with specific nasal consonants, such as M or N, or to hum in order to generate the reference sound. The patch in used to record facial bone/skin vibration during phonation by the subject. The nasal bone vibration signals recorded by the patch are then compared with the map of nasal consonants and their respective nasal bone/skin vibration pattern under normal and pathological conditions. This may then be used to identify any characteristic dips or patterns in nasal resonance, which may then be used to derive the presence of a blocked or open sinus ostium.

Figure 5 shows a method 500 for determining a physiological measure of a sinus. As described above, information on the resonant frequency ranges of different sinuses can be used to detect acoustic property of the different sinuses, which may be then used to detect a physiological measure of the sinus.

The method begins in step 510, where patch described above with reference to Figure 3 is placed over the face of a subject such that it covers the underlying sinuses. In step 520, the subject generates a reference sound, for example by speaking words with specific nasal consonants, such as M or N, or to humming.

In step 530, the facial bone vibration signals are calculated for both sides of the subject's face based on the vibration signals detected by the patch.

In step 540, it is determined whether the sinus ostium is open or blocked. The may be performed, for example, according to the method described above with reference to Figure 4. As explained above, the sinus ostium is a critical factor, which determines the resonant frequency of a sinus. Therefore, based on whether the sinus ostium is open or blocked, different workflows may be followed in order to accurately derive a physiological measure from the facial bone vibration signals.

If, in step 540, it is determined that the sinus ostium is open, the method proceeds to step 550, where the subject, or an actuator integrated into the patch, sequentially generates vibrations at different frequencies (depending on the sinus under consideration). For example, the sinuses may be insonified using frequency sweeps in the range of 200 Hz to 4 kHz.

In step 560, the responsive vibrations occurring in the bone overlying a sinus may be measured through the sensors in the patch and in step 570, the resonant frequency of the sinus is determined based on the obtained vibration signals. Resonance may be detected by comparting the duration and intensity of the facial bone's vibration for each input frequency. The input frequency with maximum duration and intensity of bone vibration denotes the resonant frequency of the underlying sinus.

As explained above reference charts of fundamental frequency can be created for each of the sinuses, based on the Equation-1, for normal and pathological conditions with an open sinus ostium. The detected resonant frequency of the sinus may then be compared with a fundamental resonant frequency (or range) of the same sinus expected under normal conditions with an open sinus ostium in step 580.

If the detected resonant frequency of a sinus is beyond expected normal range, the method proceeds to step 590, wherein it is determined that there is an abnormality present in the particular sinus, such as sinusitis, sinus polyps, a tumor, and the like.

If the detected resonant frequency of a sinus is within the expected normal range, the method proceeds to step 600, wherein it is determined that there is no abnormality present in the particular sinus.

The reference charts of fundamental frequencies of the sinus, which may be based on experimental data or machine learning-based models, may then be used to detect physiological measures of the given sinus.

If, in step 540, it is determined that the sinus ostium is blocked, the method proceeds to step 610 where the subject, or an actuator integrated into the patch, sequentially generates vibrations at different frequencies (depending on the sinus under consideration). For example, the sinuses may be insonified using frequency sweeps in the range of 200 Hz to 4 kHz.

In step 620, the responsive vibrations occurring in the bone overlying a sinus may be measured through the sensors in the patch and in step 630, the resonant frequency of the sinus is determined based on the obtained vibration signals.

As explained above, reference charts of fundamental frequency may be created for each of the sinuses based on the Equation-1, for normal and pathological conditions with a blocked sinus ostium.

In step 640, the detected resonance frequency for the sinus may be compared with a fundamental resonant frequency, or fundamental range of resonant frequencies, of the same sinus expected with a blocked sinus ostium.

If the detected resonance frequency of a sinus is beyond expected normal range, the method proceeds to step 650, not forming part of a method in accordance with the invention, wherein it is determined that there is an abnormality present in the particular sinus, such as sinusitis, sinus polyps, a tumor, and the like.

If the detected resonance frequency of a sinus is within the expected normal range, the method proceeds to step 660, not forming part of a method in accordance with the invention, wherein it is determined that there is no abnormality present in the particular sinus other than the blockage of the sinus ostium.

The reference charts of fundamental frequencies of the sinus, which may be based on experimental data or machine learning-based models, may then be used to detect physiological measures of the given sinus.

Further, the methods described above may include an investigation of a fluid within a sinus. In other words, the method may include deriving a further physiological measure of the sinus of the subject based on the comparison, wherein the further physiological measure comprises a measure of a fluid within the sinus.

In order to obtain a measure of a fluid within the sinus, the facial bone vibration signals may comprise a first subset of signals acquired when the head of the subject is maintained in an upright position and a second subset of signals acquired when the head of the subject is maintained at an angle to the upright position. The first subset of signals and the second subset of signals may then be compared in order to derive a measure of the fluid within the sinus.

During sinusitis, sinuses may become filled with mucus. The fluid in the sinus moves under the effect of gravity, which movement may be used to both detect the presence of fluid in a sinus cavity and to assess the properties of said fluid.

By way of example, in practice, the patch described above with reference to Figure 3, is placed over the face of a subject such that it covers the underlying sinuses.

When the subject's head is maintained in an upright position, the fluid in a sinus gravitates to the floor of the sinus. By an upright position, it is meant that the head of the subject is in line with an axis, along which gravity acts, defined perpendicular to the ground.

The patch is used to determine a resonant frequency, or a range of resonant frequencies, over a sinus in the upright position as described above. Based on the level of the fluid in the sinus, the different sensors within the patch may detect resonance at different frequencies. For example, the patch sensors above the fluid level may detect different frequency compared to sensors below the fluid level due to the damping of resonance by the fluid.

The subject may then tilt their head in such that the head of the subject is maintained at an angle to the upright position. The patch may then be used to determine the resonant frequency of the same sinus in each tilted position.

The resonant frequencies detected before, during and after the head tilt may then be compared to detect any change in resonant frequency as a function of the head tilt angle.

In the case of a normal sinus, without any fluid accumulation, there would not be any significant change in resonant frequency based on head position; whereas, in the case of fluid accumulation, there would be a significant change in resonant frequency as a function of the head position.

As different fluids in a sinus (watery discharge or thick discharge) possess different viscosities, they will take different lengths of time to move within the sinus in response to a change in head position. Therefore, time taken for the resonant frequency of a sinus to change may also be used to detect the type of fluid within a sinus.

Any appropriate method, such as a video camera, an accelerometer, a gyroscope, and the like, may be used to detect and quantify the magnitude of the angle of the head to the upright position.

In addition, sinuses undergo a variety of physiological changes, for example during the course of sinusitis, such as: a secretor phase, marked by profuse fluid secretions; the inflammation of the mucous membrane; thick mucus secretion; nose/ostium blockage; and the like. Each of these factors may impact the resonant frequency and amplitude of sinus vibrations.

Therefore, periodic or continuous monitoring of the resonant frequency of a sinus and/or the intensity of vibration of a sinus as described above, may be used to monitor the status of a sinus over a period of time. This information may also be used to detect early onset or recovery from sinusitis, based on earlier measurements from the same subject as a reference.

A trend of resonant frequency and/or intensity of vibration of a sinus may also be used to differentiate between acute and chronic sinusitis. In the case of acute sinusitis, it is expected that after an episode of sinusitis is over, the resonant frequency of a sinus should shift towards the natural resonant frequency of a sinus; whereas, in the case of chronic sinusitis the resonant frequency of a sinus may be outside of a normal range as long as the sinus pathology is present.

It will be understood from the description above that there are various methods for analyzing the vibration signal. These different approaches may be used alone or in combination.

In summary, these approaches involve:

(i) comparing facial skin or bone vibrations with pre-obtained reference signals to detect sinus blockage. The bones may be bones covering the wall of the sinus or nasal bones.

(ii) detecting conduction properties of the sinus by using head tilt adjustment, and by measuring resonant frequencies at different head tilt positions.

(iii) looking for changes in characteristics between the two sides of the face.

(iv) looking for resonant frequencies to identify individual sinuses.

(v) detecting an open or closed ostium state and then using a patch vibration generator to sweep frequencies to make a further assessment of the sinus pathology.

(vi) looking for trends in resonant frequencies over time.

These methods may be applied based on sounds generated by vocalization of the subj ect, but in embodiments not forming part of the present invention the sounds may be generated by external sources.

When comparison is made to reference signals, these may be signals obtained from other subjects who perform the same vocalizations or have had the same external sounds applied, but instead the reference signals may be based on physiological modelling.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

**1.** A (300) device for detecting potential sinus pathologies, comprising:

a patch (310), comprising one or more vibration sensing devices (320), configured to be placed on facial bones overlying a sinus of a subject, wherein the patch is configured to obtain facial bone vibration signals generated due to vibrations in facial bones surrounding the sinus, wherein characteristics of the facial bone vibration signals are representative of condition of the sinus; and

a processing subsystem operationally coupled to the patch,
wherein the processing subsystem is configured to:

receive the facial bone vibration signals from the patch during vocalization of sounds by the subject, resulting in resonant frequency vibrations of the facial bones; and

determine a physiological measure of the sinus of the subject based on characteristics of the facial bone vibration signals received during said vocalization of sounds by the subject, wherein the facial bone vibration signals comprise the resonant frequency vibrations.

**2.** A device (300) as claimed in claim 1, wherein the patch additionally comprises a vibrator configured to vibrate the facial bones at a frequency resulting in resonant frequency vibrations of the facial bones, wherein the facial bone vibration signals comprise the resonant frequency vibrations.

**3.** A device (300) as claimed in any of claims 1 to 2, wherein a size and a shape of the patch is dependent on an age, geographical region, target category of sinus, or combinations thereof.

4. A device (300) as claimed in any of claims 1 to 3, wherein the processing subsystem is configured to determine the physiological measure of the sinus by:

obtaining a plurality of reference sinus vibration signals corresponding to a plurality of categories of sinuses, wherein a reference sinus vibration signal corresponding to a category of sinus is different from another reference sinus vibration signal corresponding to another category of sinus;
comparing the facial bone vibration signals to the reference sinus vibration signals; and
deriving a physiological measure of the sinus of the subject based on the comparison.

5. A device (300) as claimed in any of claims 1 to 4, wherein the patch is configured to obtain the facial bone vibration signals comprising a first plurality of facial bone vibration signals from a left side of the face of the subject and a second plurality of facial bone vibration signals from a right side of the face of the subject, and wherein the processing subsystem is further configured to:

compare the first plurality of facial bone vibration signals to the second plurality of facial bone vibration signals;
determine a change in sinus resonance between the left side of the face and the right side of the face based on the comparison; and
if the change in sinus resonance exceeds a predetermined threshold, indicating the presence of an asymmetrical physiological sinus status.

6. A device (300) as claimed in any of claims 1 to 5, wherein the one or more vibration sensing devices comprises an inertia measurement unit (IMU) sensor, a microphone, an optical based sensor, or a combination thereof.

7. A device (300) as claimed in any of claims 1 to 6, wherein the processing subsystem is further configured to determine a category of sinus based on the resonant frequency of the facial bone vibration signals.

8. A device (300) as claimed in any of claims 1 to 7, the patch is configured to obtain the facial bone vibration signals comprising a third plurality of facial bone vibration signals acquired when the head of the subject is maintained in an upright position and a fourth plurality of facial bone vibration signals acquired when the head of the subject is maintained at an angle to the upright position, and wherein the processing subsystem is further configured to:

compare the third plurality of facial bone vibration signals to the fourth plurality of facial bone vibration signals; and
derive a further physiological measure of the sinus of the subject based on the comparison.

9. A device (300) as claimed in any of claims 1 to 8, wherein the patch further comprises a positioning sensor adapted to determine a position of the patch on the face of the subject, and wherein the system further comprises a user interface, wherein the processor is adapted to generate a user guidance signal for adjusting a position of the patch based on the position determined by the positioning sensor.

10. A computer-implemented method (200) for determining a physiological measure of a sinus of a subject, wherein said physiological measure may be used for detecting potential sinus pathologies, the method comprising:

obtaining (210) facial bone vibration signals from a patch (310), comprising one or more vibration sensing devices (320) placed on facial bones overlying the sinus of the subject, the facial bone vibration signals being generated due to vibrations in facial bones surrounding the sinus and generated due to vocalization of sounds by the subject resulting in resonant frequency vibrations of the facial bones, wherein characteristics of the facial bone vibration signals are representative of condition of the sinus and the facial bone vibration signals comprise the resonant frequency vibrations; and
determining (220), using a processing subsystem operationally coupled to the patch, a physiological measure of the sinus of the subject based on characteristics of the facial bone vibration signals.

11. A method as claimed in claim 10, wherein the method further comprises:

obtaining the facial bone vibration signals comprising a plurality of a first plurality of facial bone vibration signals from a left side of the face of the subject and a second plurality of facial bone vibration signals from a right side of the face of the subject;

comparing the first plurality of facial bone vibration signals to the second plurality of facial bone vibration signals;
determining a change in sinus resonance between the left side of the face and the right side of the face based on the comparison; and
if the change in sinus resonance exceeds a predetermined threshold, indicating the presence of an asymmetrical physiological sinus status.

12. A method as claimed in any of claims 10 to 11, wherein the method further comprises:

obtaining the facial bone vibration signals comprising a third plurality of facial bone vibration signals acquired when the head of the subject is maintained in an upright position and a fourth plurality of facial bone vibration signals acquired when the head of the subject is maintained at an angle to the upright position;
comparing the third plurality of facial bone vibration signals to the fourth plurality of facial bone vibration signals; and
deriving a further physiological measure of the sinus of the subject based on the comparison.

13. A computer program comprising computer program code means which is adapted, when said computer program is run on a computer, to implement the method of any of claims 10 to 12.

**Patentansprüche**

1. Eine Vorrichtung (300) zum Nachweis potenzieller Sinuspathologien, umfassend:

Ein Pflaster (310), das eine oder mehrere Schwingungsmessgeräte (320) umfasst, die so konfiguriert sind, dass sie auf Gesichtsknochen platziert werden, die über einem Sinus eines Patienten liegen, wobei das Pflaster so konfiguriert ist, dass es Schwingungssignale des Gesichtsknochens erhält, die durch Schwingungen in den Gesichtsknochen, die den Sinus umgeben, erzeugt werden; wobei die Merkmale der Schwingungssignale des Gesichtsknochens repräsentativ für den Zustand des Sinus sind, und
ein Verarbeitungs-Teilsystem, das operativ mit dem Pflaster gekoppelt ist,
wobei das Verarbeitungs-Teilsystem konfiguriert ist zum:

Empfangen, während der Vokalisierung von Geräuschen durch den Patienten, der Schwingungssignale des Gesichtsknochens vom Pflaster, was zu resonanten Frequenzschwingungen der Gesichtsknochen führt; und
Bestimmen eines physiologischen Maßes für den Sinus des Patienten, basierend auf den Merkmalen der Gesichtsknochen-Schwingungssignale, die während der besagten Vokalisierung von Geräuschen vom Patienten empfangen werden, wobei die Gesichtsknochen-Schwingungssignale die Resonanzfrequenz-Schwingungen umfassen.

2. Vorrichtung (300) nach Anspruch 1, **dadurch gekennzeichnet, dass** das Pflaster zusätzlich einen Vibrator aufweist, der konfiguriert ist, um die Gesichtsknochen mit einer Frequenz vibrieren zu lassen, die zu Resonanzfrequenz-Schwingungen der Gesichtsknochen führt, wobei die Schwingungssignale des Gesichtsknochens die Resonanzfrequenz-Schwingungen umfassen.

3. Vorrichtung (300) nach einem der Ansprüche 1 bis 2, **dadurch gekennzeichnet, dass** Größe und Form des Pflasters von einem Alter, einer geografischen Region, einer Zielkategorie des Sinus oder Kombinationen davon abhängig sind.

4. Vorrichtung (300) nach einem der Ansprüche 1 bis 3, **dadurch gekennzeichnet, dass** das Verarbeitungs-Teilsystem konfiguriert ist, das physiologische Maß des Sinus zu bestimmen durch:

Erhalten einer Mehrzahl von Sinus-Referenzschwingungssignalen, die einer Mehrzahl von Sinuskategorien entsprechen, wobei ein Referenz-Sinusschwingungssignal, das einer Sinuskategorie entspricht, sich von einem anderen Referenz-Sinusschwingungssignal, das einer anderen Sinuskategorie entspricht, unterscheidet;
Vergleich der Schwingungssignale des Gesichtsknochens mit den Referenz-Sinusschwingungssignalen; und
Ableiten eines physiologischen Maßes des Sinus des Patienten anhand des Vergleichs.

5. Vorrichtung (300) nach einem der Ansprüche 1 bis 4, wobei das Pflaster so konfiguriert ist, dass es die Gesichtsknochen-Schwingungssignale erhält, die eine erste Vielzahl von Gesichtsknochen-Schwingungssignalen von einer linken Seite des Gesichts des Patienten und eine zweite Vielzahl von Gesichtsknochen-Schwingungssignalen von einer rechten Seite des Gesichts des Patienten umfassen, und wobei das Verarbeitungs-Teilsystem weiter konfiguriert ist zum: Vergleichen der ersten Vielzahl der Schwingungssignale des Gesichtsknochens mit der zweiten Vielzahl der Schwingungssignale des Gesichtsknochens;

Bestimmen, anhand des Vergleichs, einer Änderung der Sinusresonanz zwischen der linken und der rechten Seite des Gesichts; und

Hinweisen, wenn die Änderung der Sinusresonanz einen vorher festgelegten Schwellenwert überschreitet, auf das Vorhandensein eines asymmetrischen physiologischen Sinusstatus.

6. Vorrichtung (300) nach einem der Ansprüche 1 bis 5, **dadurch gekennzeichnet, dass** das eine oder die mehreren Schwingungsmessgeräte ein Trägheitsmessgerät (IMU) Sensor, ein Mikrofon, einen optisch basierten Sensor oder eine Kombination davon umfassen.

7. Vorrichtung (300) nach einem der Ansprüche 1 bis 6, **dadurch gekennzeichnet, dass** das Verarbeitungs Teilsystem weiter konfiguriert ist, um eine Sinuskategorie basierend auf der Resonanzfrequenz der Schwingungssignale des Gesichtsknochens zu bestimmen.

8. Vorrichtung (300) nach einem der Ansprüche 1 bis 7, das Pflaster ist so konfiguriert, dass es die Schwingungssignale des Gesichtsknochens erhält, die eine dritte Vielzahl von Schwingungssignalen des Gesichtsknochens umfassen, die erfasst werden, wenn der Kopf des Patienten aufrecht gehalten wird, und eine vierte Vielzahl von Schwingungssignalen des Gesichtsknochens, die erfasst werden, wenn der Kopf des Patienten in einem Winkel zur aufrechten Position gehalten wird, und wobei das Verarbeitungs-Teilsystem weiter konfiguriert ist zum:

Vergleichen der dritten Vielzahl der Schwingungssignale des Gesichtsknochens mit der vierten Vielzahl der Schwingungssignale des Gesichtsknochens;

und Ableiten auf der Grundlage des Vergleichs eines weiteren physiologischen Maßes für den Sinus des Patienten.

9. Vorrichtung (300) nach einem der Ansprüche 1 bis 8, **dadurch gekennzeichnet, dass** das Pflaster ferner einen Positionierungssensor aufweist, der dazu geeignet ist, eine Position des Pflasters auf dem Gesicht des Patienten zu bestimmen, und wobei das System ferner eine Benutzerschnittstelle aufweist, wobei der Prozessor dazu angepasst ist, ein Benutzerführungssignal zum Einstellen einer Position des Pflasters basierend auf der durch den Positionierungssensor bestimmten Position zu erzeugen.

10. Ein durch Computer verwirklichtes Verfahren (200) zur Bestimmung eines physiologischen Maßes eines Sinus eines Patienten, wobei das physiologische Maß zum Nachweis potenzieller Sinuspathologien verwendet werden kann, wobei das Verfahren Folgendes umfasst:

Erhalten von (210) Schwingungssignalen des Gesichtsknochens von einem Pflaster (310), das eine oder mehrere Schwingungsmessgeräte (320) umfasst, die auf den Gesichtsknochen oberhalb der Sinus des Patienten angebracht sind,

die Schwingungssignale der Gesichtsknochen werden durch Schwingungen in den Gesichtsknochen erzeugt, die den Sinus umgeben, und durch die Stimmwiedergabe der Geräusche durch den Patienten erzeugt, was zu resonanten Frequenzschwingungen der Gesichtsknochen führt; wobei die Merkmale der Schwingungssignale des Gesichtsknochens repräsentativ für den Zustand des Sinus sind und die Schwingungssignale des Gesichtsknochens die resonanten Frequenzschwingungen umfassen, und

Bestimmen (220), unter Verwendung eines mit dem Pflaster operativ gekoppelten Verarbeitungs-Teilsystems, eines physiologischen Maßes des Sinus des Patienten basierend auf den Merkmalen der Schwingungssignale des Gesichtsknochens.

11. Verfahren nach Anspruch 10, **dadurch gekennzeichnet, dass** das Verfahren ferner umfasst:

Erhalten der Schwingungssignale des Gesichtsknochens, die eine Vielzahl von Schwingungssignalen des Gesichtsknochens von einer linken Seite des Gesichtsknochens und eine zweite Vielzahl von Schwingungssignalen des Gesichtsknochens von einer rechten Seite des Gesichtsknochens umfassen;

Vergleich der ersten Vielzahl von Gesichts-Knochen-Schwingungssignalen mit der zweiten Vielzahl von Gesichts-Knochen-Schwingungssignalen;
Bestimmen einer Änderung der Sinusresonanz zwischen der linken und der rechten Seite des Gesichts anhand des Vergleichs; und
Hinweisen, wenn die Änderung der Sinusresonanz einen vorher festgelegten Schwellenwert überschreitet, auf das Vorhandensein eines asymmetrischen physiologischen Sinusstatus.

12. Verfahren nach einem der Ansprüche 10 bis 11, **dadurch gekennzeichnet, dass** das Verfahren weiter umfasst:

Erhalten der Schwingungssignale des Gesichtsknochens, die eine dritte Vielzahl von Schwingungssignalen des Gesichtsknochens umfassen, die bei aufrechter Position des Kopfes des Patienten erfasst werden, und einer vierten Vielzahl von Schwingungssignalen des Gesichtsknochens, die erfasst werden, wenn der Kopf des Patienten in einem Winkel zur aufrechten Position gehalten wird;
Vergleichen der dritten Vielzahl von Gesichts-Knochen-Schwingungssignalen mit der vierten Vielzahl von Schwingungssignalen des Gesichtsknochens, und Ableiten eines weiteren physiologischen Maßes des Sinus des Patienten anhand des Vergleichs.

13. Computerprogramm, das Computerprogrammcode-Mittel umfasst, die angepasst sind, wenn das Computerprogramm auf einem Computer ausgeführt wird, das Verfahren nach einem der Ansprüche 10 bis 12 durchzuführen.

**Revendications**

1. Dispositif (300) de détection des pathologies sinusales potentielles, comprenant:

un patch (310), comprenant un ou plusieurs dispositifs de détection des vibrations (320), configuré pour être placé sur les os du visage recouvrant un sinus d'un sujet, dans lequel le patch est configuré pour obtenir des signaux de vibration des os du visage générés par les vibrations des os du visage entourant le sinus, dans lequel les caractéristiques des signaux de vibration de l'os du visage sont représentatives de l'état du sinus; et
un sous-système de traitement couplé de manière opérationnelle au patch, dans lequel le sous-système de traitement est configuré pour:

recevoir les signaux de vibration des os du visage du patch pendant la vocalisation des sons par le sujet, ce qui entraîne des vibrations à fréquence de résonance des os du visage; et
déterminer une mesure physiologique du sinus du sujet sur la base des caractéristiques des signaux de vibration des os du visage reçus pendant la vocalisation des sons par le sujet, les signaux de vibration des os du visage comprenant les vibrations de fréquence de résonance.

2. Dispositif (300) selon la revendication 1, dans lequel le patch comprend en outre un vibrateur configuré pour faire vibrer les os du visage à une fréquence entraînant des vibrations de fréquence de résonance des os du visage, les signaux de vibration des os du visage comprenant les vibrations de fréquence de résonance.

3. Dispositif (300) selon l'une des revendications 1 à 2, dans lequel la taille et la forme du patch dépendent de l'âge, de la région géographique, de la catégorie de sinus visée ou d'une combinaison de ceux-ci.

4. Dispositif (300) selon l'une des revendications 1 à 3, dans lequel le sous-système de traitement est configuré pour déterminer la mesure physiologique du sinus en:

obtenant une pluralité de signaux vibratoires sinusaux de référence correspondant à une pluralité de catégories de sinus, dans lequel un signal de vibration du sinus de référence correspondant à une catégorie de sinus est différent d'un autre signal de vibration du sinus de référence correspondant à une autre catégorie de sinus;
comparant les signaux de vibration de l'os du visage aux signaux de vibration du sinus de référence; et
déduisant une mesure physiologique du sinus du sujet sur la base de la comparaison.

5. Dispositif (300) selon l'une des revendications 1 à 4, dans lequel le patch est configuré pour obtenir les signaux de vibration des os du visage comprenant une première pluralité de signaux de vibration des os du visage provenant d'un côté gauche du visage du sujet et une seconde pluralité de signaux de vibration des os du visage provenant d'un côté droit du visage du sujet, et dans lequel le sous-système de traitement est en outre configuré pour:

comparer la première pluralité de signaux de vibration des os du visage à la seconde pluralité de signaux de vibration des os du visage;

déterminer un changement dans la résonance sinusale entre le côté gauche du visage et le côté droit du visage sur la base de la comparaison; et si la variation de la résonance sinusale dépasse un seuil prédéterminé, indiquer la présence d'un état du sinus physiologique asymétrique.

6. Dispositif (300) selon l'une des revendications 1 à 5, dans lequel le ou les dispositifs de détection des vibrations comprennent un capteur d'unité de mesure d'inertie (IMU), un microphone, un capteur optique ou une combinaison de ceux-ci.

7. A Dispositif (300) selon l'une des revendications 1 à 6, dans lequel le sous-système de traitement est en outre configuré pour déterminer une catégorie de sinus sur la base de la fréquence de résonance des signaux de vibration de l'os du visage.

8. Dispositif (300) selon l'une des revendications 1 à 7, le patch est configuré pour obtenir les signaux de vibration des os du visage comprenant une troisième pluralité de signaux de vibration des os du visage acquis lorsque la tête du sujet est maintenue en position verticale et une quatrième pluralité de signaux de vibration des os du visage acquis lorsque la tête du sujet est maintenue à un angle par rapport à la position verticale, et dans lequel le sous-système de traitement est en outre configuré pour:

comparer la troisième pluralité de signaux de vibration de l'os du visage à la quatrième pluralité de signaux de vibration de l'os du visage; et déduire une autre mesure physiologique du sinus du sujet sur la base de la comparaison.

9. Dispositif (300) selon l'une des revendications 1 à 8, dans lequel le patch comprend en outre un capteur de positionnement adapté pour déterminer une position du patch sur le visage du sujet, et dans lequel le système comprend en outre une interface utilisateur, dans laquelle le processeur est adapté pour générer un signal de guidage de l'utilisateur afin d'ajuster une position du patch sur la base de la position déterminée par le capteur de positionnement.

10. Méthode mise en œuvre par ordinateur (200) pour déterminer une mesure physiologique d'un sinus d'un sujet, dans laquelle cette mesure physiologique peut être utilisée pour détecter des pathologies sinusales potentielles, la méthode comprenant les étapes consistant à:

obtenir (210) des signaux de vibration des os du visage à partir d'un patch (310), comprenant un ou plusieurs dispositifs de détection de vibration (320) placés sur les os du visage recouvrant le sinus du sujet, les signaux de vibration des os du visage étant générés par les vibrations des os du visage entourant le sinus et générés par la vocalisation de sons par le sujet entraînant des vibrations à fréquence de résonance des os du visage, les caractéristiques des signaux de vibration des os du visage étant représentatives de l'état du sinus et les signaux de vibration des os du visage comprenant les vibrations à fréquence de résonance; et déterminer (220), à l'aide d'un sous-système de traitement couplé de manière opérationnelle au patch, une mesure physiologique du sinus du sujet sur la base des caractéristiques des signaux de vibration des os du visage.

11. Méthode selon la revendication 10, dans laquelle la méthode comprend en outre:

obtenir les signaux de vibration des os du visage comprenant une pluralité de signaux de vibration des os du visage provenant du côté gauche du visage du sujet et une seconde pluralité de signaux de vibration des os du visage provenant du côté droit du visage du sujet;

comparer la première pluralité de signaux de vibration de l'os du visage à la seconde pluralité de signaux de vibration de l'os du visage;

déterminer une modification de la résonance sinusale entre le côté gauche du visage et le côté droit du visage sur la base de la comparaison; et

si la modification de la résonance sinusale dépasse un seuil prédéterminé, indiquant la présence d'un état du sinus physiologique asymétrique.

12. Méthode selon l'une des revendications 10 à 11, dans laquelle la méthode comprend en outre les étapes consistant à:

obtenir des signaux de vibration des os du visage comprenant une troisième pluralité de signaux de vibration des os du visage acquis lorsque la tête du sujet est maintenue en position verticale et une quatrième pluralité de signaux de vibration des os du visage acquis lorsque la tête du sujet est maintenue à un angle par rapport à la position verticale;

comparer la troisième pluralité de signaux de vibration des os du visage à la quatrième pluralité de signaux de vibration des os du visage; et

déduire une autre mesure physiologique du sinus du sujet sur la base de la comparaison.

13. Programme informatique comprenant un moyen de code de programme informatique qui est adapté, lorsque ledit programme informatique est exécuté sur un ordinateur, pour mettre en oeuvre la méthode de l'une des revendications 10 à 12.

FIG. 1

Obtain facial bone vibration signals generated
due to vibrations in facial bones surrounding
the sinus

210

Determining a physiological measure of the
sinus of the subject based on characteristics of
the facia bone vibration signals

220

200

FIG. 2

FIG. 3

410 — Place patch over sinus

420 — Subject generates reference sound

430 — Compute subject sinus vibration signals on both sides of face

440 —

No

Indicate asymmetrical status

450

Are both sides comparable?

Yes

No sinus ostium blockage

490

460 —

No

No

Frequency pattern changes?

480

Vibration intensity less than threshold?

Yes

Yes

Sinus ostium blockage

470

400

FIG. 4

510 — Place patch over sinus

520 — Subject generates reference sound

530 — Compute subject sinus vibration signals on both sides of face

540 — Is sinus ostium (SO) open?

610 — Insonify sinus with a frequency sweep ← No

620 — Measure sinus vibration signal intensity and frequency

630 — Determine resonant frequency (RF)

640 — RF in normal range?

660 — Blocked SO, no sinus pathology ← Yes

Blocked SO and sinus pathology — 650 ← No

550 — Insonify sinus with a frequency sweep ← Yes

560 — Measure sinus vibration signal intensity and frequency

570 — Determine resonant frequency (RF)

580 — RF in normal range?

600 — Open SO, no sinus pathology ← Yes

Open SO and sinus pathology — 590 ← No

500

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005154301 A
- RU 169125
- SU 1648379
- US 20040210135 A

### Non-patent literature cited in the description

- **J. NOVIS et al.** A Diagnostic Dilemma: Chronic Sinusitis Diagnosed by Non-Otolaryngologists. *Int Forum Allergy Rhinol,* May 2016, vol. 6 (5), 486-490
- **J. BRAMLEY et al.** Productivity losses related to the common cold. *J. Occup. Environ. Med.,* September 2002, vol. 44 (9), 822-829
- **N. BHATTACHARYYA.** Clinical and symptom criteria for the accurate diagnosis of chronic rhinosinusitis. *Laryngoscope,* July 2006, vol. 116 (7), 1-22
- **T. PUHAKKA et al.** Validity of Ultrasonography in Diagnosis of Acute Maxillary Sinusitis. *Arch Otolaryngol Head Neck Surg,* December 2000, vol. 126 (12), 1482-1486
- **M. HAVEL et al.** Eliminating paranasal sinus resonance and its effects on acoustic properties of the nasal tract. *Logoped Phoniatr Vocol,* 2016, vol. 41 (1), 33-40
- **T. KOYAMA.** Experimental study on the resonance of paranasal sinus. *Nippon Jibiinkoka Gakkai Kaiho,* 1966, vol. 69 (6), 1177-1191
- **M. NARAGHI et al.** Nitric oxide: a new concept in chronic sinusitis pathogenesis. *Am J Otolaryngol,* October 2007, vol. 28 (5), 334-337
- **F. C. CHEN et al.** Facial bone vibration in resonant voice production. *J Voice,* September 2014, vol. 28 (5), 596-602
- **E. M.-L. YIU et al.** Vibratory and perceptual measurement of resonant voice. *J Voice,* September 2012, vol. 26 (5), 675.e13-19